(19) Europäisches Patentamt
European Patent Office
Office européen des brevets

(11) **EP 3 019 241 B1**

(12) **EUROPEAN PATENT SPECIFICATION**

(45) Date of publication and mention
of the grant of the patent:
**30.05.2018 Bulletin 2018/22**

(21) Application number: **14739923.2**

(22) Date of filing: **09.07.2014**

(51) Int Cl.:
*A61P 25/00* *(2006.01)*          *A61K 38/48* *(2006.01)*
*A61K 39/08* *(2006.01)*

(86) International application number:
**PCT/GB2014/052101**

(87) International publication number:
**WO 2015/004464 (15.01.2015 Gazette 2015/02)**

(54) **SUPPRESSION OF ITCH**

UNTERDRÜCKUNG VON JUCKREIZ

SUPPRESSION DES DÉMANGEAISONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2013 GB 201312295**

(43) Date of publication of application:
**18.05.2016 Bulletin 2016/20**

(73) Proprietor: **Ipsen Bioinnovation Limited Abingdon Oxfordshire OX14 3YS (GB)**

(72) Inventor: **FOSTER, Keith Abingdon Oxfordshire OX14 3YS (GB)**

(74) Representative: **MacLean, Martin Robert et al Mathys & Squire LLP The Shard 32 London Bridge Street London SE1 9SG (GB)**

(56) References cited:
**WO-A1-2012/156743       US-A1- 2011 091 437 US-A1- 2012 230 975**

• **P. SIKAND ET AL:** "BAM8-22 Peptide Produces Itch and Nociceptive Sensations in Humans Independent of Histamine Release", JOURNAL OF NEUROSCIENCE, vol. 31, no. 20, 18 May 2011 (2011-05-18), pages 7563-7567, XP055152432, ISSN: 0270-6474, DOI: 10.1523/JNEUROSCI.1192-11.2011

• **ROBERT H. LAMOTTE ET AL:** "Sensory neurons and circuits mediating itch", NATURE REVIEWS NEUROSCIENCE, vol. 15, no. 1, 20 December 2013 (2013-12-20), pages 19-31, XP055152428, ISSN: 1471-003X, DOI: 10.1038/nrn3641

• **QIN LIU ET AL:** "Sensory Neuron-Specific GPCR Mrgprs Are Itch Receptors Mediating Chloroquine-Induced Pruritus", CELL, vol. 139, no. 7, 1 December 2009 (2009-12-01), pages 1353-1365, XP055152433, ISSN: 0092-8674, DOI: 10.1016/j.cell.2009.11.034

• **LIANG HAN ET AL:** "A subpopulation of nociceptors specifically linked to itch", NATURE NEUROSCIENCE, vol. 16, no. 2, 23 December 2012 (2012-12-23), pages 174-182, XP055152492, ISSN: 1097-6256, DOI: 10.1038/nn.3289

• **FOSTER KEITH ET AL:** "Targeted Secretion Inhibitors-Innovative Protein Therapeutics", TOXINS, vol. 2, no. 12, 3 December 2010 (2010-12-03), pages 2795-2815, XP055152496, DOI: 10.3390/toxins2122795

• **LIANG HAN ET AL:** "Itch Mechanisms and Circuits", ANNUAL REVIEW OF BIOPHYSICS, vol. 43, no. 1, 6 May 2014 (2014-05-06), pages 331-355, XP055152607, ISSN: 1936-122X, DOI: 10.1146/annurev-biophys-051013-022826

• **Q. Liu ET AL:** "The Distinct Roles of Two GPCRs, MrgprC11 and PAR2, in Itch and Hyperalgesia", Science Signaling, vol. 4, no. 181, 12 July 2011 (2011-07-12), pages ra45-ra45, XP55315020, US ISSN: 1945-0877, DOI: 10.1126/scisignal.2001925

**(Cont. next page)**

Remarks:
The file contains technical information submitted after the application was filed and not included in this specification

Remarks:
The file contains technical information submitted after the application was filed and not included in this specification

**Description**

The present invention provides methods and compositions for the suppression or treatment of itch.

Itch, medically known as pruritus, has been defined as an "unpleasant skin sensation that elicits the desire or reflex to scratch". Patients with severe itch often find it difficult to lead a normal life due to associated discomfort and psychological disturbances, such as depression or sleep deprivation. Itch can also be a debilitating condition that accompanies numerous skin, systemic and nervous system disorders.

Itch is hypothesised to have evolved to protect animals against small clinging threats such as insects and plant spines that would not be effectively removed by the withdrawal response, associated with sensing pain. A close evolutionary relationship is evident in the overlap between itch and pain neurons: both are mediated via free nerve endings of unmyelinated C-type nerve fibres (nociceptors). There are a myriad of mediators capable of stimulating these nerves, such as biogenic amines, alkaloids, proteases, and peptides. Nociceptors detect the mediators through their peripheral axons, and send signals to the spinal cord to produce, for example, itch perceptions in the brain. For clinicians, the overlap between itch and pain means that the same neurological diseases that can cause neuropathic pain can also cause neuropathic itch. However, there are differences. Most treatments effective for pain are not effective for itch. Notably, some analgesics such as opioid pain relievers (e.g. morphine) even cause, or worsen itch.

Recent studies have begun to delineate characteristics of itch circuitry separate from pain. It has been suggested that itch is characterised as relayed by a subset of nociceptors, referred to as 'pruriceptors' or itch-specific nerve fibres, located at the dermoepidermal junction and within the epidermis. Certain G-protein coupled receptors (GPCRs) specifically expressed in the sensory neurons in the dorsal root ganglia (DRG) or in the trigeminal nerve are thought to play an essential role in itch-unique sensations. This group of receptors have been recently identified as unique to itch (i.e. they are not involved in the pain circuitry), and present potential therapeutic targets towards suppressing itch.

According to the Global Burden of Disease (GBD) study conducted in 2000, 4% of the population (approximately 280 million) suffer with conditions associated with itch. Presently anti-histamine and corticosteroid drugs can relieve some types of acute itch and a small percentage of chronic itch types. However they do not treat the majority of cases of chronic itch resulting from renal diseases and liver diseases, cancers, as well as skin diseases such as atopic dermatitis. In addition, anti-histamines only counteract histamine-dependent itch (usually triggered by an allergic stimulus). However, histamine-independent itch accounts for the majority of itch cases.

Severe scratching can injure the skin making it prone to infection, with serious implications for immunocompromised patients (where itch may be secondary to a condition or drug reaction). Furthermore injured and infected skin due to itch often intensifies the "itch-scratch-itch cycle" (the cycle of which an itch demands a scratch and the scratch further deepens an itch). Other techniques for relieving itch recommended by physicians include: phototherapy, wearing loose cotton clothing; taking short frequent baths in warm water and maintaining a cool environment with a 30% to 40% humidity level. However these preventatives measures are not always possible to maintain. Hence, there remains a need in the art for new medicaments for suppressing or treating itch.

This need is addressed by the present invention, which solves one or more of the above-mentioned problems.

**Summary of the Invention**

The present invention addresses one or more of the above-mentioned problems by providing a fusion protein for use in suppression or treatment of itch in a subject (e.g. patient), said fusion protein comprising:

(i) a non-cytotoxic protease, which protease is capable of cleaving a SNARE (soluble N-ethylmaleimide sensitive factor attachment protein receptor) protein in an itch-specific DRG neuron or a pruriceptor;

(ii) a Targeting Moiety (TM) that is capable of binding to a Binding Site on an itch-specific DRG neuron or a pruriceptor, which Binding Site is capable of undergoing endocytosis to be incorporated into an endosome within the itchspecific DRG neuron or a pruriceptor, and wherein said itch-specific DRG neuron or a pruriceptor expresses said SNARE protein; and

(iii) a translocation domain that is capable of translocating the protease from within an endosome, across the endosomal membrane and into the cytosol of the itch-specific DRG neuron or a pruriceptor;

with the proviso that the polypeptide is not a clostridial neurotoxin (holotoxin) molecule.

As claimed, the Binding Site is a Mas-related G protein coupled receptor (Mrgpr).

Also described is a corresponding method for suppressing or treating itch, said method comprising administering a therapeutically effective amount of a polypeptide of the present invention to a patient.

The invention also provides a polypeptide comprising:

(i) a non-cytotoxic protease, which protease is capable of cleaving a SNARE protein in an itch-specific DRG neuron or a pruriceptor;

(ii) a Targeting Moiety (TM) that is capable of binding to a Mas-related G protein-coupled receptor (Mrgpr) on the itch-specific DRG neuron or a pruriceptor, which Mrgpr is capable of undergoing endocytosis to be incorporated into an endosome within the itch-specific DRG neuron or a pruriceptor, and wherein said itch-specific DRG neuron or a pruriceptor expresses said SNARE protein; and

(iii) a translocation domain that is capable of translocating the protease from within an endosome, across the endosomal membrane and into the cytosol of the itch-specific DRG neuron or a pruriceptor;

with the proviso that the polypeptide is not a clostridial neurotoxin (holotoxin) molecule, and wherein the TM is selected from the group consisting of: a Melanocyte Stimulating Hormone peptide (MSH), neuropeptides terminating in Y-G or Y-amide, a chloroquine (CQ), a peptide comprising SLIGRL-NH$_2$, histamine, serotonin, or truncations or peptide analogues thereof.

## Detailed Description of the Invention

The polypeptide of the present invention is not a naturally-occurring clostridial neurotoxin molecule (also known as clostridial holotoxin). Clostridial holotoxin is one of the most lethal neurotoxins known to man, and, as such, has significant limitations as a therapeutic molecule. Also, in the context of suppressing itch, clostridial holotoxin is associated with undesirable off-site targeting, i.e. targeting of cells other than nerve cells of the itch pathway.

In use, a polypeptide of the invention binds to a receptor on an itch-specific DRG neuron or a pruriceptor. The translocation component effects transport of the protease component into the cytosol of the itch-specific DRG neuron or a pruriceptor. Finally, once inside, the protease inhibits the exocytic fusion process of the itch-specific DRG neuron or a pruriceptor by cleaving SNARE protein present in the cytosol of the itch-specific DRG neuron or a pruriceptor. Thus, by inactivating the exocytic fusion apparatus of the itch-specific DRG neuron or a pruriceptor, the polypeptide of the invention inhibits secretion of neurotransmitter therefrom. Accordingly, the polypeptide of the invention reduces the level of the neurotransmitter sent to the spinal cord from the itch-specific DRG neuron or a pruriceptor and hence is capable of suppressing or treating itch. In one embodiment the neurotransmitter is acetylcholine. In a preferred embodiment the neurotransmitter is glutamate. In another preferred embodiment, the neurotransmitter is gastrin releasing protein (GRP).

The polypeptides of the present invention provide a distinct advantage over other therapeutics in that they have the potential to inhibit the secretion from a specific target cell, the itch-specific DRG neuron or a pruriceptor. In contrast, other proposed therapeutic agents seek to reduce itch by attempting to use an antagonist to the receptor that mediates the effect. However, the present invention provides a means of specifically blocking neurotransmitter secretion from its site of production.

The principal target cell of the present invention is an itch-specific DRG neuron or a pruriceptor. DRG cells are located along the vertebral column by the spine, and are an expression site of GPCRs such as MrgprX1, MrgprA3 or MrgprC11, which have been identified as itch-specific receptors.

The fusion proteins of the present invention generally demonstrate a reduced binding affinity (in the region of up to 10 to 100-fold) for target cells when compared with the corresponding 'free' TM (i.e. the isolated TM *per* se). However, despite this observation, the fusion proteins of the present invention surprisingly demonstrate good efficacy. This can be attributed to two principal features. First, the non-cytotoxic protease component is catalytic - thus, the therapeutic effect of a few such molecules is rapidly amplified within a target cell. Secondly, the receptors present on the target cells need only act as a gateway for entry of the therapeutic, and need not necessarily be stimulated to a level required in order to achieve a ligand-receptor mediated pharmacological response. Accordingly, the fusion proteins of the present invention may be administered at a dosage that is lower than would be employed for other types of therapeutic molecules, which are typically administered at high microgram to milligram (even up to hundreds of milligram) quantities. In contrast, the fusion proteins of the present invention may be administered at much lower dosages, typically at least 10-fold lower, and more typically at 100-fold lower.

## The non-cytotoxic protease

The biologically active component of the TSI polypeptides of the present invention is a non-cytotoxic protease. Thus, once delivered into the cytosol of a target cell, the non-cytotoxic protease component effects SNARE cleavage within the desired target cell. Since SNARE proteins are an essential component of the secretory process within mammalian cells, proteolytic inactivation thereof inhibits/ suppresses secretion from said cells.

Non-cytotoxic proteases are a discrete class of molecules that do not kill cells; instead, they act by inhibiting cellular processes other than protein synthesis. Non-cytotoxic proteases are produced by a variety of higher organisms (e.g. plants, and animals) - an example of such a higher organism is the Brazilian scorpion. In addition, non-cytotoxic proteases are produced by a variety of microorganisms, notably bacteria such as Clostridium sp. and Neisseria sp.

Clostridial neurotoxins represent a major group of non-cytotoxic toxin molecules, and comprise two polypeptide chains joined together by a disulphide bond. The two chains are termed the heavy chain (H-chain), which has a molecular mass of approximately 100 kDa, and the light chain (L-chain), which has a molecular mass of approximately 50 kDa. It is the L-chain, which possesses a protease function and exhibits high substrate specificity for vesicle or plasma membrane associated SNARE proteins involved in the exocytic process (e.g. syntaxin, SNAP and synaptobrevin (or VAMP)). These substrates are essential components of a cell's secretory machinery.

Neisseria sp., most notably from the species *N. gonorrhoeae,* produce functionally similar non-cytotoxic toxin molecules. An example of such a non-cytotoxic protease is IgA protease (see WO99/58571). Similar IgA proteases are produced by streptococci, such as *Streptococcus pneumoniae.*

Thus, in one embodiment the non-cytotoxic protease of the present invention may be a clostridial neurotoxin protease or an IgA protease (see, for example, WO 99/032272). Another example of non-cytotoxic proteases is a scorpion venom protease, such as those from the venom of the Brazilian scorpion *Tityus serrulatus,* or the protease antarease (see, for example, WO 2011/022357).

**The targeting moiety (TM)**

Turning now to the Targeting Moiety (TM) component of the present invention, it is this component that binds the polypeptide of the present invention to an itch-specific DRG neuron or a pruriceptor. The TM is preferably a peptide.

In use, a polypeptide of the invention binds to an itch-specific DRG neuron or a pruriceptor. Thereafter, the translocation component of the polypeptide effects transport of the protease component into the cytosol of the itch-specific DRG neuron or a pruriceptor. Finally, once inside, the protease inhibits the exocytic fusion process of the itch-specific DRG neuron or a pruriceptor by cleaving SNARE protein present in the cytosol of the itch-specific DRG neuron or a pruriceptor. Thus, by inactivating the exocytic fusion apparatus of the itch-specific DRG neuron or a pruriceptor, the polypeptide of the invention inhibits secretion of neurotransmitter therefrom. Accordingly, the polypeptide of the invention reduces the transmission of itch-sensation signals via the spinal cord to the brain, and hence is capable of suppressing or treating itch.

The TM binds to a Binding Site on the itch-specific DRG neuron or a pruriceptor, thereby providing selectivity of the polypeptide to this species of target cell over other cells. In this regard, preferred TM embodiments of the present invention include antibodies (e.g. monoclonal antibodies, antibody fragments such as Fab, F(ab)'$_2$, Fv, ScFv, etc., and antibody domains peptides), as well as binding scaffolds, which bind to the receptors identified below. Accordingly, the polypeptides of present invention may include commercially available antibodies or binding scaffolds, which have been designed to achieve specific binding to the target cell or receptor in question.

Alternatively, preferred TMs include biogenic amines, alkaloids, proteases, peptide ligands, and neuropeptides.

A TM of the present invention binds to an itch-specific DRG neuron or a pruriceptor. As claimed, a TM of the polypeptide of the present invention binds to a G-protein coupled receptor (GPCR) on an itch-specific DRG neuron or a pruriceptor, selected from the group comprising a Mas-related G-protein receptor (e.g. a MrgprX1, MrgprA3 or MrgprC11).

In one embodiment, the TM is selected from a bovine adrenal medulla peptide (e.g. BAM$_{8-22}$), a melanocyte stimulating hormone peptide (e.g. γ2-MSH), a neuropeptide terminating in a RF/Y-G or a RF/Y-amide (e.g. NPFF or NPAF), a peptide comprising SLIGRL-NH$_2$, alkaloids (e.g. chloroquine), biogenic amines (capsaicin, histamine, serotonin, cortistatin), as well as truncations and peptide analogues thereof.

In one embodiment the TM of the polypeptide of the present invention binds to a receptor on an itch-specific DRG neuron or a pruriceptor selected from the group comprising: MrgprX1, MrgprA3, or MrgprC11. All of these receptors are expressed on itch-specific DRG neurons or a pruriceptors.

In one embodiment, the TM is selected from: BAM$_{8-22}$, γ2-MSH, NPFF, NPAF, SLIGRL-NH$_2$, chloroquine, capsaicin, histamine, serotonin, cortistatin, as well as truncations and peptide analogues thereof.

As claimed, a TM of the polypeptide of the present invention binds to an Mrgpr receptor, and preferably MrgprX, MrgprA or MrgprC. In one embodiment, the polypeptide of the present invention binds to MrgpX1 or MrgprA3 or MrgprC11. By way of example, suitable TMs include: BAM$_{8-22}$, chloroquine, γ2-MSH, neuropeptides terminating in RF/Y-G or an RF/Y-amide (e.g. NPFF or NPAF), capsaicin, histamine, serotonin, or cortistatin. These TMs are preferred for binding to Mrgprs.

In a preferred embodiment, the TM binds to the MrgprX receptor, preferably MrgprX1; also preferred is that the TM is BAM$_{8-22}$ or a truncation or peptide analogue thereof.

**The translocation domain**

The translocation component of the present invention enables translocation of the non-cytotoxic protease (or fragment thereof) into the target cell so that functional expression of protease activity occurs within the cytosol of the target cell. The translocation component is preferably capable of forming ion-permeable pores in lipid membranes (e.g. endosomal membranes) under conditions of low pH. The translocation component may be obtained from a microbial protein source, for example a bacterial or viral protein source. Hence, in one embodiment, the translocation component comprises or consists of a translocation domain of an enzyme, such as a bacterial toxin. In another embodiment, the translocation domain comprises or consists of the translocation domain of a viral protein. In one embodiment, the translocation component of the present invention may comprise or consist of a clostridial neurotoxin H-chain or a fragment thereof such as the $H_N$ domain (or a translocating fragment thereof) of a clostridial neurotoxin.

**Polypeptide preparation**

The polypeptides of the present invention comprise 3 principal components: a 'bioactive' (i.e. a non-cytotoxic protease); a TM; and a translocation domain. The general technology associated with the preparation of such fusion proteins is often referred to as re-targeted toxin technology. By way of exemplification, we refer to: WO94/21300; WO96/33273; WO98/07864; WO00/10598; WO01/21213; WO06/059093; WO00/62814; WO00/04926; WO93/15766; WO00/61192; and WO99/58571.

In more detail, the TM component of the present invention may be fused to either the protease component or the translocation component of the present invention. Said fusion is preferably by way of a covalent bond, for example either a direct covalent bond or via a spacer/ linker molecule. The protease component and the translocation component are preferably linked together via a covalent bond, for example either a direct covalent bond or via a spacer/ linker molecule. Suitable spacer/ linked molecules are well known in the art, and typically comprise an amino acid-based sequence of between 5 and 40, preferably between 10 and 30 amino acid residues in length.

In use, the polypeptides have a di-chain conformation, wherein the protease component and the translocation component are linked together, preferably via a disulphide bond.

The polypeptides of the present invention may be prepared by conventional chemical conjugation techniques, which are well known to a skilled person. By way of example, reference is made to Hermanson, G.T. (1996), Bioconjugate techniques, Academic Press, and to Wong, S.S. (1991), Chemistry of protein conjugation and cross-linking, CRC Press, Nagy et al., PNAS 95 p1794-99 (1998). Further detailed methodologies for attaching synthetic TMs to a polypeptide of the present invention are provided in, for example, EP0257742.

Alternatively, the polypeptides may be prepared by recombinant preparation of a single polypeptide fusion protein (see, for example, WO98/07864). This technique is based on the *in vivo* bacterial mechanism by which native clostridial neurotoxin (i.e. holotoxin) is prepared, and results in a fusion protein having the following 'simplified' structural arrangement:

$$NH_2 - [\text{protease component}] - [\text{translocation component}] - [TM] - COOH$$

According to WO98/07864, the TM is placed towards the C-terminal end of the fusion protein. The fusion protein is then activated by treatment with a protease, which cleaves at a site between the protease component and the translocation component. A di-chain protein is thus produced, comprising the protease component as a single polypeptide chain covalently attached (via a disulphide bridge) to another single polypeptide chain containing the translocation component plus TM.

Alternatively, according to WO06/059093, the TM component of the fusion protein is located towards the middle of the linear fusion protein sequence, between the protease cleavage site and the translocation component. This ensures that the TM is attached to the translocation domain (i.e. as occurs with native clostridial holotoxin), though in this case the two components are reversed in order *vis-à-vis* native holotoxin. Subsequent cleavage at the protease cleavage site exposes the N-terminal portion of the TM, and provides the di-chain polypeptide fusion protein.

A further alternative is the 'split ligand' presentation of the TM component of the fusion protein. Here the TM component, which acts as a ligand, has both a free N-terminal domain and a free C-terminal domain. Thus, the TM is capable of interacting with the binding site (e.g. a receptor or acceptor) on a target cell via an interaction between an N-terminal portion of the targeting moiety and a domain of the binding site. Alternatively, the TM is capable of an interaction between the C-terminal portion of the targeting moiety and a domain of a binding site. Or, the TM is capable of a dual interaction, wherein an N-terminal portion of the targeting moiety interacts with a domain of the binding site and a C-terminal portion of the targeting moiety interacts with a domain of a binding site. In this latter embodiment, the N- and C-terminal portions of the TM may bind to the same or different domains of a binding site, and/ or may bind to domains on different binding sites. Further information regarding this arrangement may be found in WO2012156743.

The above-mentioned protease cleavage sequence(s) may be introduced (and/ or any inherent cleavage sequence removed) at the DNA level by conventional means, such as by site-directed mutagenesis. Screening to confirm the presence of cleavage sequences may be performed manually or with the assistance of computer software (e.g. the MapDraw program by DNASTAR, Inc.). Whilst any protease cleavage site may be employed (i.e. clostridial, or non-clostridial), the following are preferred:

| | |
|---|---|
| Enterokinase | (DDDDK↓) (SEQ ID NO: 1) |
| Factor Xa | (IEGR↓ / IDGR↓) (SEQ ID NOS 2 and 3) |
| TEV(Tobacco Etch virus) | (ENLYFQ↓G) (SEQ ID NO: 4) |
| Thrombin | (LVPR↓GS) (SEQ ID NO: 5) |
| PreScission | (LEVLFQ↓GP). (SEQ ID NO: 6) |

Additional protease cleavage sites include recognition sequences that are cleaved by a non-cytotoxic protease, for example by a clostridial neurotoxin. These include the SNARE (e.g. SNAP-25, syntaxin, VAMP) protein recognition sequences that are cleaved by non-cytotoxic proteases such as clostridial neurotoxins. Particular examples are provided in US2007/0166332.

Also embraced by the term protease cleavage site is an intein, which is a self-cleaving sequence. The self-splicing reaction is controllable, for example by varying the concentration of reducing agent present. The above-mentioned 'activation' cleavage sites may also be employed as a 'destructive' cleavage site (discussed below) should one be incorporated into a polypeptide of the present invention.

In a preferred embodiment, the fusion protein of the present invention may comprise one or more N-terminal and/ or C-terminal located purification tags. Whilst any purification tag may be employed, the following are preferred:

His-tag (e.g. 6 x histidine) (SEQ ID NO: 7), preferably as a C-terminal and/ or N-terminal tag
MBP-tag (maltose binding protein), preferably as an N-terminal tag
GST-tag (glutathione-S-transferase), preferably as an N-terminal tag
His-MBP-tag, preferably as an N-terminal tag
GST-MBP-tag, preferably as an N-terminal tag
Thioredoxin-tag, preferably as an N-terminal tag
CBD-tag (Chitin Binding Domain), preferably as an N-terminal tag.

One or more peptide spacer/ linker molecules may be included in the fusion protein. For example, a peptide spacer may be employed between a purification tag and the rest of the fusion protein molecule.

The present invention also provides a DNA sequence that encodes a fusion protein protein of the claimed invention.

The DNA sequence may be prepared as part of a DNA vector, wherein the vector comprises a promoter and terminator.

In a preferred embodiment, the vector has a promoter selected from:

| Promoter | Induction Agent | Typical Induction Condition |
|---|---|---|
| Tac (hybrid) | IPTG | 0.2 mM (0.05-2.0mM) |
| AraBAD | L-arabinose | 0.2% (0.002-0.4%) |
| T7-*lac* operator | IPTG | 0.2 mM (0.05-2.0mM) |

The DNA construct of the present invention is preferably designed *in silico,* and then synthesised by conventional DNA synthesis techniques.

The above-mentioned DNA sequence information is optionally modified for codon-biasing according to the ultimate host cell (e.g. *E. coli*) expression system that is to be employed.

The DNA backbone is preferably screened for any inherent nucleic acid sequence, which when transcribed and translated would produce an amino acid sequence corresponding to the protease cleavage site encoded by the second peptide-coding sequence. This screening may be performed manually or with the assistance of computer software (e.g. the MapDraw program by DNASTAR, Inc.).

Reference to "suppressing" and "treating" as used herein, means to provide a therapeutic benefit to a subject. It includes, for example, administering a fusion protein as defined herein to prevent or lessen the severity of itch.

A further aspect of the invention as described is a method of preventing or suppressing itch, wherein said method comprises administering to said subject a therapeutically effective amount of a fusion protein comprising:

(i) a non-cytotoxic protease, which protease is capable of cleaving a SNARE protein in an itch-specific DRG neuron or a pruriceptor;
(ii) a Targeting Moiety (TM) that is capable of binding to a Binding Site on an itch-specific DRG neuron or a pruriceptor, which Binding Site is capable of undergoing endocytosis to be incorporated into an endosome within the itch-specific DRG neuron or a pruriceptor, and wherein said itch-specific DRG neuron or a pruriceptor expresses said SNARE protein; and

(iii) a translocation domain that is capable of translocating the protease from within an endosome, across the endosomal membrane and into the cytosol of the itch-specific DRG neuron or a pruriceptor;

with the proviso that the polypeptide is not a clostridial neurotoxin (holotoxin) molecule.

The fusion proteins of the present invention may include a destructive protease cleavage site, which is susceptible to cleavage (by a local protease) in the event that the fusion protein might migrate to an off-site location. This approach helps to minimise the risk of off-site targeting. Thus, the fusion proteins of the present invention may be designed to include one or more destructive cleavage sites, for example, as described in WO 2010/094905 and WO 2002/44199.

**Polypeptide delivery**

In use, the present invention may employ a pharmaceutical composition, comprising a polypeptide, together with at least one component selected from a pharmaceutically acceptable carrier, excipient, adjuvant, propellant and/ or salt.

The polypeptides of the present invention may be formulated for oral, parenteral, continuous infusion, implant, inhalation or topical application. Compositions suitable for injection may be in the form of solutions, suspensions or emulsions, or dry powders which are dissolved or suspended in a suitable vehicle prior to use.

Local delivery means may include an oral or gastric delivery. In this regard, formulations in enteric-coated capsules or other particulate systems such as microspheres can be used. Local administration to the duodenum via laparoscopic surgery is also possible. Other examples of local delivery may also include transdermal delivery (via an adhesive patch).

The preferred route of administration is selected from: systemic, oral, laparoscopic and/ or localised injection.

In the case of formulations for injection, it is optional to include a pharmaceutically active substance to assist retention at or reduce removal of the polypeptide from the site of administration. One example of such a pharmaceutically active substance is a vasoconstrictor such as adrenaline. Such a formulation confers the advantage of increasing the residence time of polypeptide following administration and thus increasing and/or enhancing its effect.

The dosage ranges for administration of the polypeptides of the present invention are those to produce the desired therapeutic effect. It will be appreciated that the dosage range required depends on the precise nature of the polypeptide or composition, the route of administration, the nature of the formulation, the age of the patient, the nature, extent or severity of the patient's condition, contraindications, if any, and the judgement of the attending physician. Variations in these dosage levels can be adjusted using standard empirical routines for optimisation.

Suitable daily dosages (per kg weight of patient) are in the range 0.0001-1 mg/kg, preferably 0.0001-0.5 mg/kg, more preferably 0.002-0.5 mg/kg, and particularly preferably 0.004-0.5 mg/kg. The unit dosage can vary from less than 1 microgram to 30mg, but typically will be in the region of 0.01 to 1 mg per dose, which may be administered daily or preferably less frequently, such as weekly or six monthly.

A particularly preferred dosing regimen is based on 2.5 ng of polypeptide as the 1X dose. In this regard, preferred dosages are in the range 1X-100X (i.e. 2.5-250 ng).

Fluid dosage forms are typically prepared utilising the polypeptide and a pyrogen-free sterile vehicle. The polypeptide, depending on the vehicle and concentration used, can be either dissolved or suspended in the vehicle. In preparing solutions the polypeptide can be dissolved in the vehicle, the solution being made isotonic if necessary by addition of sodium chloride and sterilised by filtration through a sterile filter using aseptic techniques before filling into suitable sterile vials or ampoules and sealing. Alternatively, if solution stability is adequate, the solution in its sealed containers may be sterilised by autoclaving. Advantageously additives such as buffering, solubilising, stabilising, preservative or bactericidal, suspending or emulsifying agents and or local anaesthetic agents may be dissolved in the vehicle.

Dry powders, which are dissolved or suspended in a suitable vehicle prior to use, may be prepared by filling pre-sterilised ingredients into a sterile container using aseptic technique in a sterile area. Alternatively the ingredients may be dissolved into suitable containers using aseptic technique in a sterile area. The product is then freeze dried and the containers are sealed aseptically.

Parenteral suspensions, suitable for intramuscular, subcutaneous or intradermal injection, are prepared in substantially the same manner, except that the sterile components are suspended in the sterile vehicle, instead of being dissolved as sterilisation cannot be accomplished by filtration. The components may be isolated in a sterile state or alternatively it may be sterilised after isolation, e.g. by gamma irradiation.

Advantageously, a suspending agent for example polyvinylpyrrolidone is included in the composition/s to facilitate uniform distribution of the components.

Administration in accordance with the present invention may take advantage of a variety of delivery technologies including microparticle encapsulation, viral delivery systems or high-pressure aerosol impingement.

**Definitions Section**

Targeting Moiety (TM) means any chemical structure that functionally interacts with a Binding Site to cause a physical association between the polypeptide of the invention and the surface of a target cell. In the context of the present invention, the target cell is an itch-specific DRG neuron or a pruriceptor. The term TM embraces any molecule (i.e. a naturally occurring molecule, or a chemically/physically modified variant thereof) that is capable of binding to a Binding Site on the target cell, which Binding Site is capable of internalisation (e.g. endosome formation) - also referred to as receptor-mediated endocytosis. The TM may possess an endosomal membrane translocation function, in which case separate TM and Translocation Domain components need not be present in an agent of the present invention. Throughout the preceding description, specific TMs have been described. Reference to said TMs in the context of the claimed medical uses is merely exemplary, and the present invention embrace all variants and derivatives thereof, which retain the basic binding (i.e. targeting) ability of the exemplified TMs.

A TM according to the present invention includes antibodies (e.g. antibody fragments) and binding scaffolds; especially commercially available antibodies/ fragments and scaffolds designed for the purpose of binding (e.g. specifically) to target cells.

Protein scaffolds represent a new generation of universal binding frameworks to complement the expanding repertoire of therapeutic monoclonal antibodies and derivatives such as scFvs, Fab molecules, dAbs (single-domain antibodies), camelids, diabodies and minibodies, each of which may be employed as a TM of the present invention. Scaffold systems create or modify known protein recognition domains either through creation of novel scaffolds or modification of known protein binding domains. Such scaffolds include but are not limited to:

(i) protein A based scaffolds - affibodies (Nord, K. et al 1997 "Binding proteins selected from combinatorial libraries of an alpha-helical bacterial receptor domain". Nat Biotechnol 15, 772-777);
(ii) lipocalin based scaffolds - anticalins (Skerra 2008 "Alternative binding proteins: anticalins - harnessing the structural plasticity of the lipocalin ligand pocket to engineer novel binding activities". FEBS J. 275:2677-83);
(iii) fibronectin based scaffolds - adnectin (Dineen et al 2008 "The Adnectin CT-322 is a novel VEGF receptor 2 inhibitor that decreases tumour burden in an orthotropic mouse model of pancreatic cancer". BMC Cancer 8:352);
(iv) avimers (Silverman et al 2005 "Multivalent avimer proteins evolved by exon shuffling of a family of human receptor domains". Nat Biotechnol 23:1556-61);
(v) ankyrin based scaffolds - darpins (Zahnd et al 2006 "Selection and characterization of Her2 binding-designed ankyrin repeat proteins". J Biol Chem. 281:35167-75); and
(vi) centyrin scaffolds - based on a protein fold that has significant structural homology to Ig domains with loops that are analogous to CDRs. Ig domains are a common module in human proteins and have been widely applied as alternative scaffold proteins.

Binding scaffolds can be used to target particular cell types via interaction with specific cell surface proteins, receptors or other cell surface epitopes such as sugar groups. Such modified scaffolds can be engineered onto recombinant non-cytotoxic protease based polypeptides of the present invention.

The TM of the present invention binds (preferably specifically binds) to the itch-specific DRG neuron or a pruriceptor target cell in question. As claimed, the TM binds to a Mrgpr on said DRG neuron or pruriceptor target cell. The term "specifically binds" preferably means that a given TM binds to the target cell with a binding affinity (Ka) of $10^6$ $M^{-1}$ or greater, preferably $10^7$ $M^{-1}$ or greater, more preferably $10^8$ $M^{-1}$ or greater, and most preferably, $10^9$ $M^{-1}$ or greater. The term "specifically binds" can also mean that a given TM binds to a given receptor, Mas-related G protein coupled receptor (e.g. MrgprX1 or $MrgprA_{1-3}$ or MrgprC11) with a binding affinity (Ka) of $10^6$ $M^{-1}$ or greater, preferably $10^7$ $M^{-1}$ or greater, more preferably $10^8$ $M^{-1}$ or greater, and most preferably, $10^9$ $M^{-1}$ or greater.

Reference to TM in the present specification embraces fragments and variants thereof, which retain the ability to bind to the target cell in question. By way of example, a variant may have at least 80%, preferably at least 90%, more preferably at least 95%, and most preferably at least 97 or at least 99% amino acid sequence homology with the reference TM (e.g. any SEQ ID NO presented in the present specification, which defines a TM). Thus, a variant may include one or more analogues of an amino acid (e.g. an unnatural amino acid), or a substituted linkage. Also, by way of example, the term fragment, when used in relation to a TM, means a peptide having at least ten, preferably at least twenty, more preferably at least thirty, and most preferably at least forty amino acid residues of the reference TM. The term fragment also relates to the above-mentioned variants. Thus, by way of example, a fragment of the present invention may comprise a peptide sequence having at least 10, 20, 30 30 or 40 amino acids, wherein the peptide sequence has at least 80% sequence homology over a corresponding peptide sequence (of contiguous) amino acids of the reference peptide.

It is routine to confirm that a TM binds to the selected target cell or Mrgpr. For example, a simple radioactive displacement experiment may be employed in which tissue or cells representative of a target cell in question are exposed to labelled (e.g. tritiated) TM in the presence of an excess of unlabelled TM. In such an experiment, the relative proportions of non-specific and specific binding may be assessed, thereby allowing confirmation that the TM binds to the target cell. Optionally, the assay may include one or more binding antagonists, and the assay may further comprise observing a loss of TM binding. Examples of this type of experiment can be found in Hulme, E.C. (1990), Receptor-binding studies, a brief outline, pp. 303-311, In Receptor biochemistry, A Practical Approach, Ed. E.C. Hulme, Oxford University Press.

In the context of the present invention, reference to a peptide TM embraces peptide analogues thereof, so long as the analogue binds to the same receptor as the corresponding 'reference' TM.

The fusion proteins (also referred to herein as polypeptides) of the present invention may lack a functional $H_C$ or $H_{CC}$ domain of a clostridial neurotoxin. In one embodiment, the polypeptides lack the last 50 C-terminal amino acids of a clostridial neurotoxin holotoxin. In another embodiment, the polypeptides lack the last 100, 150, 200, 250, or 300 C-terminal amino acid residues of a clostridial neurotoxin holotoxin. Alternatively, the $H_C$ binding activity may be negated/ reduced by mutagenesis - by way of example, referring to BoNT/ A for convenience, modification of one or two amino acid residue mutations (W1266 to L and Y1267 to F) in the ganglioside binding pocket causes the $H_C$ region to lose its receptor binding function. Analogous mutations may be made to non-serotype A clostridial peptide components, e.g. a construct based on botulinum B with mutations (W1262 to L and Y1263 to F) or botulinum E (W1224 to L and Y1225 to F). Other mutations to the active site achieve the same ablation of $H_C$ receptor binding activity, e.g. Y1267S in botulinum type A toxin and the corresponding highly conserved residue in the other clostridial neurotoxins. Details of this and other mutations are described in Rummel et al (2004) (Molecular Microbiol. 51:631-634).

The $H_C$ peptide of a native clostridial neurotoxin comprises approximately 400-440 amino acid residues, and consists of two functionally distinct domains of approximately 25kDa each, namely the N-terminal region (commonly referred to as the $H_{CN}$ peptide or domain) and the C-terminal region (commonly referred to as the $H_{CC}$ peptide or domain). Moreover, it has been well documented that the C-terminal region ($H_{CC}$), which constitutes the C-terminal 160-200 amino acid residues, is responsible for binding of a clostridial neurotoxin to its natural cell receptors, namely to nerve terminals at the neuromuscular junction. Thus, reference throughout this specification to a clostridial heavy-chain lacking a functional heavy chain $H_C$ peptide (or domain) such that the heavy-chain is incapable of binding to cell surface receptors to which a native clostridial neurotoxin binds means that the clostridial heavy-chain simply lacks a functional $H_{CC}$ peptide. In other words, the $H_{CC}$ peptide region is either partially or wholly deleted, or otherwise modified (e.g. through conventional chemical or proteolytic treatment) to inactivate its native binding ability for nerve terminals at the neuromuscular junction.

Thus, in one embodiment, a clostridial $H_N$ peptide of the present invention may lack part of a C-terminal peptide portion ($H_{CC}$) of a clostridial neurotoxin and thus lacks the $H_C$ binding function of native clostridial neurotoxin. By way of example, in one embodiment, the C-terminally extended clostridial $H_N$ peptide lacks the C-terminal 40 amino acid residues, or the C-terminal 60 amino acid residues, or the C-terminal 80 amino acid residues, or the C-terminal 100 amino acid residues, or the C-terminal 120 amino acid residues, or the C-terminal 140 amino acid residues, or the C-terminal 150 amino acid residues, or the C-terminal 160 amino acid residues of a clostridial neurotoxin heavy-chain. In another embodiment, the clostridial $H_N$ peptide of the present invention may lack the entire C-terminal peptide portion ($H_{CC}$) of a clostridial neurotoxin and thus lacks the $H_C$ binding function of native clostridial neurotoxin. By way of example, in one embodiment, the clostridial $H_N$ peptide lacks the C-terminal 165 amino acid residues, or the C-terminal 170 amino acid residues, or the C-terminal 175 amino acid residues, or the C-terminal 180 amino acid residues, or the C-terminal 185 amino acid residues, or the C-terminal 190 amino acid residues, or the C-terminal 195 amino acid residues of a clostridial neurotoxin heavy-chain. By way of further example, the clostridial $H_N$ peptide of the present invention lacks a clostridial $H_{CC}$ reference sequence selected from the group consisting of:

Botulinum type A neurotoxin - amino acid residues (Y1111-L1296)
Botulinum type B neurotoxin - amino acid residues (Y1098-E1291)
Botulinum type C neurotoxin - amino acid residues (Y1112-E1291)
Botulinum type D neurotoxin - amino acid residues (Y1099-E1276)
Botulinum type E neurotoxin - amino acid residues (Y1086-K1252)
Botulinum type F neurotoxin - amino acid residues (Y1106-E1274)
Botulinum type G neurotoxin - amino acid residues (Y1106-E1297)
Tetanus neurotoxin - amino acid residues (Y1128-D1315).

The above-identified reference sequences should be considered a guide as slight variations may occur according to sub-serotypes.

The protease of the present invention embraces all non-cytotoxic proteases that are capable of cleaving one or more proteins of the exocytic fusion apparatus in eukaryotic cells.

The protease of the present invention is preferably a bacterial protease (or fragment thereof). More preferably the bacterial protease is selected from the genera *Clostridium* or *Neisserial Streptococcus* (e.g. a clostridial L-chain, or a neisserial IgA protease preferably from *N. gonorrhoeae or S. pneumoniae).* Another example of non-cytotoxic proteases includes scorpion venom protease, such as those from the venom of the Brazilian scorpion *Tityus serrulatus,* or the protease antarease.

The present invention also embraces variant non-cytotoxic proteases (i.e. variants of naturally-occurring protease molecules), so long as the variant proteases still demonstrate the requisite protease activity. By way of example, a variant may have at least 70%, preferably at least 80%, more preferably at least 90%, and most preferably at least 95% or at least 98% amino acid sequence homology with a reference protease sequence. Thus, the term variant includes non-cytotoxic proteases having enhanced (or decreased) endopeptidase activity - particular mention here is made to the increased $K_{cat}/K_m$ of BoNT/A mutants Q161A, E54A, and K165L see Ahmed, S.A. (2008) Protein J. DOI 10.1007/s10930-007-9118-8.

The term fragment, when used in relation to a protease, typically means a peptide having at least 150, preferably at least 200, more preferably at least 250, and most preferably at least 300 amino acid residues of the reference protease. As with the TM 'fragment' component (discussed above), protease 'fragments' of the present invention embrace fragments of variant proteases based on a reference sequence.

The protease of the present invention preferably demonstrates a serine or metalloprotease activity (e.g. endopeptidase activity). The protease is preferably specific for a SNARE protein (e.g. SNAP-25, synaptobrevin/VAMP, or syntaxin).

Particular mention is made to the protease domains of neurotoxins, for example the protease domains of bacterial neurotoxins. Thus, the present invention embraces the use of neurotoxin domains, which occur in nature, as well as recombinantly prepared versions of said naturally-occurring neurotoxins.

Exemplary neurotoxins are produced by clostridia, and the term clostridial neurotoxin embraces neurotoxins produced by *C. tetani* (TeNT), and by *C. botulinum* (BoNT) serotypes A-G, as well as the closely related BoNT-like neurotoxins produced by *C. baratii* and *C. butyricum.* The above-mentioned abbreviations are used throughout the present specification. For example, the nomenclature BoNT/A denotes the source of neurotoxin as BoNT (serotype A). Corresponding nomenclature applies to other BoNT serotypes.

BoNTs are the most potent toxins known, with median lethal dose (LD50) values for mice ranging from 0.5 to 5 ng/kg depending on the serotype.

BoNTs are adsorbed in the gastrointestinal tract, and, after entering the general circulation, bind to the presynaptic membrane of cholinergic nerve terminals and prevent the release of their neurotransmitter acetylcholine. BoNT/B, BoNT/D, BoNT/F and BoNT/G cleave synaptobrevin/vesicle-associated membrane protein (VAMP); BoNT/C, BoNT/A and BoNT/E cleave the synaptosomal-associated protein of 25 kDa (SNAP-25); and BoNT/C cleaves syntaxin.

BoNTs share a common structure, being di-chain proteins of ~150 kDa, consisting of a heavy chain (H-chain) of ~100 kDa covalently joined by a single disulphide bond to a light chain (L-chain) of ~50 kDa. The H-chain consists of two domains, each of ~50 kDa. The C-terminal domain ($H_C$) is required for the high-affinity neuronal binding, whereas the N-terminal domain ($H_N$) is proposed to be involved in membrane translocation. The L-chain is a zinc-dependent metalloprotease responsible for the cleavage of the substrate SNARE protein.

The term L-chain fragment means a component of the L-chain of a neurotoxin, which fragment demonstrates a metalloprotease activity and is capable of proteolytically cleaving a vesicle and/or plasma membrane associated protein involved in cellular exocytosis.

Examples of suitable protease (reference) sequences include:

| | |
|---|---|
| Botulinum type A neurotoxin | - amino acid residues (1-448) |
| Botulinum type B neurotoxin | - amino acid residues (1-440) |
| Botulinum type C neurotoxin | - amino acid residues (1-441) |
| Botulinum type D neurotoxin | - amino acid residues (1-445) |
| Botulinum type E neurotoxin | - amino acid residues (1-422) |
| Botulinum type F neurotoxin | - amino acid residues (1-439) |
| Botulinum type G neurotoxin | - amino acid residues (1-441) |
| Tetanus neurotoxin | - amino acid residues (1-457) |
| IgA protease | - amino acid residues (1-959)* |
| * Pohlner, J. et al. (1987). Nature 325, pp. 458-462. | |

The above-identified reference sequence should be considered a guide as slight variations may occur according to sub-serotypes. By way of example, US 2007/0166332 cites slightly different clostridial sequences:

| Botulinum type A neurotoxin | - amino acid residues (M1-K448) |
| Botulinum type B neurotoxin | - amino acid residues (M1-K441) |
| Botulinum type C neurotoxin | - amino acid residues (M1-K449) |
| Botulinum type D neurotoxin | - amino acid residues (M1-R445) |
| Botulinum type E neurotoxin | - amino acid residues (M1-R422) |
| Botulinum type F neurotoxin | - amino acid residues (M1-K439) |
| Botulinum type G neurotoxin | - amino acid residues (M1-K446) |
| Tetanus neurotoxin | - amino acid residues (M1-A457) |

A variety of clostridial toxin fragments comprising the light chain can be useful in aspects of the present invention with the proviso that these light chain fragments can specifically target the core components of the neurotransmitter release apparatus and thus participate in executing the overall cellular mechanism whereby a clostridial toxin proteolytically cleaves a substrate. The light chains of clostridial toxins are approximately 420-460 amino acids in length and comprise an enzymatic domain. Research has shown that the entire length of a clostridial toxin light chain is not necessary for the enzymatic activity of the enzymatic domain. As a non-limiting example, the first eight amino acids of the BoNT/A light chain are not required for enzymatic activity. As another non-limiting example, the first eight amino acids of the TeNT light chain are not required for enzymatic activity. Likewise, the carboxyl-terminus of the light chain is not necessary for activity. As a non-limiting example, the last 32 amino acids of the BoNT/A light chain (residues 417-448) are not required for enzymatic activity. As another non-limiting example, the last 31 amino acids of the TeNT light chain (residues 427-457) are not required for enzymatic activity. Thus, aspects of this embodiment can include clostridial toxin light chains comprising an enzymatic domain having a length of, for example, at least 350 amino acids, at least 375 amino acids, at least 400 amino acids, at least 425 amino acids and at least 450 amino acids. Other aspects of this embodiment can include clostridial toxin light chains comprising an enzymatic domain having a length of, for example, at most 350 amino acids, at most 375 amino acids, at most 400 amino acids, at most 425 amino acids and at most 450 amino acids.

Further examples of suitable non-cytotoxic proteases are described in detail in WO 2007/106115.

In one embodiment, the non-cytotoxic protease cleaves a non-neuronal SNARE protein such as a SNAP-23 protein. In one embodiment, the non-cytotoxic protease is a modified botulinum toxin L-chain capable of cleaving SNAP-23. An example of such a modified L-chain is described by Chen and Barbieri, PNAS, vol. 106, no. 23, p9180-9184, 2009.

In one embodiment, the non-cytotoxic protease is a BoNT/A, BoNT/C or BoNT/E protease, and the preferred SNARE motif is a SNAP (e.g. SNAP 25) motif.

In another embodiment, the non-cytotoxic protease is a BoNT/B, BoNT/D, BoNT/F or BoNT/G or tetanus neurotoxin (TeNT) protease, and the preferred SNARE motif is a VAMP motif.

In another embodiment, the non-cytotoxic protease is a BoNT/C$_1$ protease, and the preferred SNARE motif is a syntaxin motif.

The non-cytotoxic proteases of the present invention recognise different cleavage site sequences and thus have slightly different cleavage specificities.

| Non-cytotoxic Protease | Cleavage site recognition sequence: P4-P3-P2-P1-↓-P1'-P2'-P3' | | | | | | |
|---|---|---|---|---|---|---|---|
| | P4 | P3 | P2 | P1 | P1' | P2' | P3' |
| BoNT/A | E | A | N | Q | R | A | T |
| BoNT/B | G | A | S | Q | F | E | T |
| BoNT/C | A | N | Q | R | A | T | K |
| BoNT/C | D | T | K | K | A | V | K |
| BoNT/D | R | D | Q | K | L | S | E |
| BoNT/E | Q | I | D | R | I | M | E |
| BoNT/F | E | R | D | Q | K | L | S |
| BoNT/G | E | T | S | A | A | K | I |
| TeNT | G | A | S | Q | F | E | T |
| IgA protease | S | T | P | P | T | P | S |
| Antarease | I | K | R | K | Y | W | W |

By way of further example, reference is made to the following recognition sequences and cleavage sites:

| Non-cytotoxic Protease | Cleavage site recognition sequence: P4-P3-P2-P1-↓-P1'-P2'-P3' | | | | | | |
|---|---|---|---|---|---|---|---|
| | P4 | P3 | P2 | P1 | P1' | P2' | P3' |
| BoNT/A | E | A | N | Q | R | A | T |
| | A | N | Q | R | A | T | K |
| | E | A | N | Q | R | A | T |
| | F | A | N | Q | R | A | T |
| | E | A | N | Q | R | A | T |
| | E | A | N | Q | R | A | I |
| | E | A | N | K | A | T | K |
| | E | A | N | K | H | A | T |
| | E | A | N | K | H | A | N |
| | | | | Q | R | | |
| | | | | K | H | | |
| BoNT/C | D | E | A | N | Q | R | A |
| | E | A | N | Q | R | A | T |
| | A | N | Q | R | A | T | K |
| | N | Q | R | A | T | K | M |
| | A | N | Q | R | A | I | K |
| | A | N | Q | R | A | H | Q |
| | D | T | K | K | A | V | K |
| | K | T | K | K | A | V | K |
| | E | T | K | K | A | I | K |
| | E | T | K | R | A | M | K |
| | D | T | K | K | A | V | R |
| | D | T | K | K | A | L | K |
| | D | T | K | K | A | M | K |
| | E | S | K | K | A | V | K |
| | E | T | K | K | A | M | K |
| | E | T | K | K | A | V | K |
| | | | | K | A | | |
| | | | | R | A | | |
| BoNT/E | Q | I | D | R | I | M | E |
| | Q | I | Q | K | I | T | E |
| | Q | I | D | R | I | V | E |
| | Q | F | D | R | I | M | D |
| | Q | F | D | R | I | M | E |
| | Q | L | D | R | I | H | D |
| | Q | I | D | R | I | M | D |
| | Q | V | D | R | I | Q | Q |
| | | | | R | I | | |
| | | | | K | I | | |
| BoNT/B | G | A | S | Q | F | E | T |
| | A | G | A | S | Q | F | E |
| | G | A | S | Q | F | E | S |
| | Q | A | S | Q | F | E | S |
| | G | A | S | Q | G | E | T |
| | G | A | S | Q | F | E | Q |

(continued)

| Non-cytotoxic Protease | Cleavage site recognition sequence: P4-P3-P2-P1-↓-P1'-P2'-P3' | | | | | | |
|---|---|---|---|---|---|---|---|
| | P4 | P3 | P2 | P1 | P1' | P2' | P3' |
| | Q | A | S | Q | F | E | A |
| | G | A | S | Q | F | Q | Q |
| | G | A | S | Q | F | E | A |
| | | | | Q | F | | |
| BoNT/D | R | D | Q | K | L | S | E |
| | R | D | Q | K | I | S | E |
| | K | D | Q | K | L | A | E |
| | | | | K | L | | |
| BoNT/F | E | R | D | Q | K | L | S |
| | V | L | E | R | D | Q | K |
| | E | R | D | Q | K | I | S |
| | E | R | D | Q | A | L | S |
| | E | K | D | Q | K | L | A |
| | | | | Q | K | | |
| BoNT/G | E | S | S | A | A | K | I |
| | E | T | S | A | A | K | I |
| | E | S | S | A | A | K | L |
| | E | T | S | A | A | K | L |
| | | | | A | A | | |
| TeNT | G | A | S | Q | F | E | T |
| | G | A | S | Q | G | E | T |
| | G | A | S | Q | F | E | Q |
| | Q | A | S | Q | F | E | A |
| | G | A | S | Q | F | E | S |
| | Q | A | S | Q | F | E | S |
| | G | A | S | Q | F | Q | Q |
| | G | A | S | Q | F | E | A |
| | | | | Q | F | | |
| IgA protease | S | T | P | P | T | P | S |
| Antarease | I | K | R | K | Y | W | W |

The polypeptides of the present invention, especially the protease component thereof, may be PEGylated - this may help to increase stability, for example duration of action of the protease component. PEGylation is particularly preferred when the protease comprises a BoNT/A, B or $C_1$ protease. PEGylation preferably includes the addition of PEG to the N-terminus of the protease component. By way of example, the N-terminus of a protease may be extended with one or more amino acid (e.g. cysteine) residues, which may be the same or different. One or more of said amino acid residues may have its own PEG molecule attached (e.g. covalently attached) thereto. An example of this technology is described in WO2007/104567.

A Translocation Domain is a molecule that enables translocation of a protease into a target cell such that a functional expression of protease activity occurs within the cytosol of the target cell. Whether any molecule (e.g. a protein or peptide) possesses the requisite translocation function of the present invention may be confirmed by any one of a number of conventional assays.

For example, Shone C. (1987) describes an *in vitro* assay employing liposomes, which are challenged with a test molecule. Presence of the requisite translocation function is confirmed by release from the liposomes of $K^+$ and/ or labelled NAD, which may be readily monitored [see Shone C. (1987) Eur. J. Biochem; vol. 167(1): pp. 175-180].

A further example is provided by Blaustein R. (1987), which describes a simple *in vitro* assay employing planar phospholipid bilayer membranes. The membranes are challenged with a test molecule and the requisite translocation function is confirmed by an increase in conductance across said membranes [see Blaustein (1987) FEBS Letts; vol. 226, no. 1: pp. 115-120].

Additional methodology to enable assessment of membrane fusion and thus identification of Translocation Domains suitable for use in the present invention are provided by Methods in Enzymology Vol 220 and 221, Membrane Fusion Techniques, Parts A and B, Academic Press 1993.

The present invention also embraces variant translocation domains, so long as the variant domains still demonstrate the requisite translocation activity. By way of example, a variant may have at least 70%, preferably at least 80%, more preferably at least 90%, and most preferably at least 95% or at least 98% amino acid sequence homology with a reference translocation domain. The term fragment, when used in relation to a translocation domain, means a peptide having at least 20, preferably at least 40, more preferably at least 80, and most preferably at least 100 amino acid residues of the reference translocation domain. In the case of a clostridial translocation domain, the fragment preferably has at least 100, preferably at least 150, more preferably at least 200, and most preferably at least 250 amino acid residues of the reference translocation domain (e.g. $H_N$ domain). As with the TM 'fragment' component (discussed above), translocation 'fragments' of the present invention embrace fragments of variant translocation domains based on the reference sequences.

The Translocation Domain is preferably capable of formation of ion-permeable pores in lipid membranes under conditions of low pH. Preferably it has been found to use only those portions of the protein molecule capable of pore-formation within the endosomal membrane.

The Translocation Domain may be obtained from a microbial protein source, in particular from a bacterial or viral protein source. Hence, in one embodiment, the Translocation Domain is a translocating domain of an enzyme, such as a bacterial toxin or viral protein.

It is well documented that certain domains of bacterial toxin molecules are capable of forming such pores. It is also known that certain translocation domains of virally expressed membrane fusion proteins are capable of forming such pores. Such domains may be employed in the present invention.

The Translocation Domain may be of a clostridial origin, such as the $H_N$ domain (or a functional component thereof). $H_N$ means a portion or fragment of the H-chain of a clostridial neurotoxin approximately equivalent to the amino-terminal half of the H-chain, or the domain corresponding to that fragment in the intact H-chain. In this regard, should it be desired to remove the $H_C$ cell-binding function, this may be done by deletion of the $H_C$ or $H_{CC}$ amino acid sequence (either at the DNA synthesis level, or at the post-synthesis level by nuclease or protease treatment). Alternatively, the $H_C$ function may be inactivated by chemical or biological treatment.

Examples of suitable (reference) Translocation Domains include:

| | |
|---|---|
| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |
| Botulinum type G neurotoxin | - amino acid residues (442-863) |
| Tetanus neurotoxin | - amino acid residues (458-879) |

The above-identified reference sequence should be considered a guide as slight variations may occur according to sub-serotypes. By way of example, US 2007/0166332 (hereby incorporated by reference thereto) cites slightly different clostridial sequences:

| | |
|---|---|
| Botulinum type A neurotoxin | - amino acid residues (A449-K871) |
| Botulinum type B neurotoxin | - amino acid residues (A442-S858) |
| Botulinum type C neurotoxin | - amino acid residues (T450-N866) |
| Botulinum type D neurotoxin | - amino acid residues (D446-N862) |
| Botulinum type E neurotoxin | - amino acid residues (K423-K845) |
| Botulinum type F neurotoxin | - amino acid residues (A440-K864) |
| Botulinum type G neurotoxin | - amino acid residues (S447-S863) |
| Tetanus neurotoxin | - amino acid residues (S458-V879) |

Further examples of suitable translocation domains are described in detail in WO 2007/106115.

In the context of the present invention, a variety of clostridial toxin $H_N$ regions comprising a translocation domain can be useful in aspects of the present invention with the proviso that these active fragments can facilitate the release of a non-cytotoxic protease (e.g. a clostridial L-chain) from intracellular vesicles into the cytoplasm of the target cell and thus participate in executing the overall cellular mechanism whereby a clostridial toxin proteolytically cleaves a substrate. The $H_N$ regions from the heavy chains of clostridial toxins are approximately 410-430 amino acids in length and comprise a translocation domain. Research has shown that the entire length of a $H_N$ region from a clostridial toxin heavy chain is not necessary for the translocating activity of the translocation domain. Thus, aspects of this embodiment can include clostridial toxin $H_N$ regions comprising a translocation domain having a length of, for example, at least 350 amino acids, at least 375 amino acids, at least 400 amino acids and at least 425 amino acids. Other aspects of this embodiment can include clostridial toxin $H_N$ regions comprising translocation domain having a length of, for example, at most 350 amino acids, at most 375 amino acids, at most 400 amino acids and at most 425 amino acids.

For further details on the genetic basis of toxin production in *Clostridium botulinum* and *C. tetani,* we refer to Henderson et al (1997) in The Clostridia: Molecular Biology and Pathogenesis, Academic press.

The term $H_N$ embraces naturally-occurring neurotoxin $H_N$ portions, and modified $H_N$ portions having amino acid sequences that do not occur in nature and/ or synthetic amino acid residues, so long as the modified $H_N$ portions still demonstrate the above-mentioned translocation function.

Alternatively, the Translocation Domain may be of a non-clostridial origin. Examples of non-clostridial (reference) Translocation Domain origins include, but not be restricted to, the translocation domain of diphtheria toxin [O=Keefe et al., Proc. Natl. Acad. Sci. USA (1992) 89, 6202-6206; Silverman et al., J. Biol. Chem. (1993) 269, 22524-22532; and London, E. (1992) Biochem. Biophys. Acta., 1112, pp.25-51*]*, the translocation domain of *Pseudomonas* exotoxin type A [Prior et al. Biochemistry (1992) 31, 3555-3559], the translocation domains of anthrax toxin [Blanke et al. Proc. Natl. Acad. Sci. USA (1996) 93, 8437-8442], a variety of fusogenic or hydrophobic peptides of translocating function [Plank et al. J. Biol. Chem. (1994) 269, 12918-12924; and Wagner et al (1992) PNAS, 89, pp.7934-7938*]*, and amphiphilic peptides [Murata et al (1992) Biochem., 31, pp. 1986-1992]. The Translocation Domain may mirror the Translocation Domain present in a naturally-occurring protein, or may include amino acid variations so long as the variations do not destroy the translocating ability of the Translocation Domain.

Particular examples of viral (reference) Translocation Domains suitable for use in the present invention include certain translocating domains of virally expressed membrane fusion proteins. For example, Wagner *et al.* (1992) and Murata *et al.* (1992) describe the translocation (i.e. membrane fusion and vesiculation) function of a number of fusogenic and amphiphilic peptides derived from the N-terminal region of influenza virus haemagglutinin. Other virally expressed membrane fusion proteins known to have the desired translocating activity are a translocating domain of a fusogenic peptide of Semliki Forest Virus (SFV), a translocating domain of vesicular stomatitis virus (VSV) glycoprotein G, a translocating domain of SER virus F protein and a translocating domain of Foamy virus envelope glycoprotein. Virally encoded Aspike proteins have particular application in the context of the present invention, for example, the E1 protein of SFV and the G protein of the G protein of VSV.

Use of the (reference) Translocation Domains listed in Table (below) includes use of sequence variants thereof. A variant may comprise one or more conservative nucleic acid substitutions and/ or nucleic acid deletions or insertions, with the proviso that the variant possesses the requisite translocating function. A variant may also comprise one or more amino acid substitutions and/ or amino acid deletions or insertions, so long as the variant possesses the requisite translocating function.

| Translocation Domain source | Amino acid residues | References |
| --- | --- | --- |
| Diphtheria toxin | 194-380 | Silverman etal., 1994, J. Biol. Chem. 269, 22524-22532 London E., 1992, Biochem. Biophys. Acta., 1113, 25-51 |
| Domain II of pseudomonas exotoxin | 405-613 | Prior et al., 1992, Biochemistry 31, 3555-3559 Kihara & Pastan, 1994, Bioconj Chem. 5, 532-538 |
| Influenza virus haemagglutinin | GLFGAIAGFIENGW EGMIDGWYG, and Variants thereof | Plank et al., 1994, J. Biol. Chem. 269, 12918-12924 Wagner et al., 1992, PNAS, 89, 7934-7938 Murata et al., 1992, Biochemistry 31, 1986-1992 |
| Semliki Forest virus fusogenic protein | Translocation domain | Kielian et al., 1996, J Cell Biol. 134(4), 863-872 |

(continued)

| Translocation Domain source | Amino acid residues | References |
|---|---|---|
| Vesicular Stomatitis virus glycoprotein G | 118-139 | Yao et al., 2003, Virology 310(2), 319-332 |
| SER virus F protein | Translocation domain | Seth et al., 2003, J Virol 77(11) 6520-6527 |
| Foamy virus envelope glycoprotein | Translocation domain | Picard-Maureau et al., 2003, J Virol. 77(8), 4722-4730 |

The polypeptides of the present invention may further comprise a translocation facilitating domain. Said domain facilitates delivery of the non-cytotoxic protease into the cytosol of the target cell and are described, for example, in WO 08/008803 and WO 08/008805.

By way of example, suitable translocation facilitating domains include an enveloped virus fusogenic peptide domain, for example, suitable fusogenic peptide domains include influenza virus fusogenic peptide domain (e.g. influenza A virus fusogenic peptide domain of 23 amino acids), alphavirus fusogenic peptide domain (e.g. Semliki Forest virus fusogenic peptide domain of 26 amino acids), vesiculovirus fusogenic peptide domain (e.g. vesicular stomatitis virus fusogenic peptide domain of 21 amino acids), respirovirus fusogenic peptide domain (e.g. Sendai virus fusogenic peptide domain of 25 amino acids), morbiliivirus fusogenic peptide domain (e.g. Canine distemper virus fusogenic peptide domain of 25 amino acids), avulavirus fusogenic peptide domain (e.g. Newcastle disease virus fusogenic peptide domain of 25 amino acids), henipavirus fusogenic peptide domain (e.g. Hendra virus fusogenic peptide domain of 25 amino acids), metapneumovirus fusogenic peptide domain (e.g. Human metapneumovirus fusogenic peptide domain of 25 amino acids) or spumavirus fusogenic peptide domain such as simian foamy virus fusogenic peptide domain; or fragments or variants thereof.

By way of further example, a translocation facilitating domain may comprise a Clostridial toxin $H_{CN}$ domain or a fragment or variant thereof. In more detail, a Clostridial toxin $H_{CN}$ translocation facilitating domain may have a length of at least 200 amino acids, at least 225 amino acids, at least 250 amino acids, at least 275 amino acids. In this regard, a Clostridial toxin $H_{CN}$ translocation facilitating domain preferably has a length of at most 200 amino acids, at most 225 amino acids, at most 250 amino acids, or at most 275 amino acids. Specific (reference) examples include:

Botulinum type A neurotoxin - amino acid residues (872-1110)
Botulinum type B neurotoxin - amino acid residues (859-1097)
Botulinum type C neurotoxin - amino acid residues (867-1111)
Botulinum type D neurotoxin - amino acid residues (863-1098)
Botulinum type E neurotoxin - amino acid residues (846-1085)
Botulinum type F neurotoxin - amino acid residues (865-1105)
Botulinum type G neurotoxin - amino acid residues (864-1105)
Tetanus neurotoxin - amino acid residues (880-1127)

The above sequence positions may vary a little according to serotype/ subtype, and further examples of suitable (reference) Clostridial toxin $H_{CN}$ domains include:

Botulinum type A neurotoxin - amino acid residues (874-1110)
Botulinum type B neurotoxin - amino acid residues (861-1097)
Botulinum type C neurotoxin - amino acid residues (869-1111)
Botulinum type D neurotoxin - amino acid residues (865-1098)
Botulinum type E neurotoxin - amino acid residues (848-1085)
Botulinum type F neurotoxin - amino acid residues (867-1105)
Botulinum type G neurotoxin - amino acid residues (866-1105)
Tetanus neurotoxin - amino acid residues (882-1127)

Any of the above-described facilitating domains may be combined with any of the previously described translocation domain peptides that are suitable for use in the present invention. Thus, by way of example, a non-clostridial facilitating domain may be combined with non-clostridial translocation domain peptide or with clostridial translocation domain peptide. Alternatively, a Clostridial toxin $H_{CN}$ translocation facilitating domain may be combined with a non-clostridial translocation domain peptide. Alternatively, a Clostridial toxin $H_{CN}$ facilitating domain may be combined or with a clostridial translocation domain peptide, examples of which include:

| | |
|---|---|
| Botulinum type A neurotoxin | - amino acid residues (449-1110) |
| Botulinum type B neurotoxin | - amino acid residues (442-1097) |
| Botulinum type C neurotoxin | - amino acid residues (450-1111) |
| Botulinum type D neurotoxin | - amino acid residues (446-1098) |
| Botulinum type E neurotoxin | - amino acid residues (423-1085) |
| Botulinum type F neurotoxin | - amino acid residues (440-1105) |
| Botulinum type G neurotoxin | - amino acid residues (447-1105) |
| Tetanus neurotoxin | - amino acid residues (458-1127) |

**Sequence homology**

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

Alignment scores for determining sequence identity

```
        A R N D C Q E G H I L K M F P S T W Y V
A  4
R -1  5
N -2  0  6
D -2 -2  1  6
C  0 -3 -3 -3  9
Q -1  1  0  0 -3  5
E -1  0  0  2 -4  2  5
G  0 -2  0 -1 -3 -2 -2  6
H -2  0  1 -1 -3  0  0 -2  8
I -1 -3 -3 -3 -1 -3 -3 -4 -3  4
L -1 -2 -3 -4 -1 -2 -3 -4 -3  2  4
K -1  2  0 -1 -3  1  1 -2 -1 -3 -2  5
M -1 -1 -2 -3 -1  0 -2 -3 -2  1  2 -1  5
F -2 -3 -3 -3 -2 -3 -3 -3 -1  0  0 -3  0  6
P -1 -2 -2 -1 -3 -1 -1 -2 -2 -3 -3 -1 -2 -4  7
S  1 -1  1  0 -1  0  0  0 -1 -2 -2  0 -1 -2 -1  4
T  0 -1  0 -1 -1 -1 -1 -2 -2 -1 -1 -1 -1 -2 -1  1  5
W -3 -3 -4 -4 -2 -2 -3 -2 -2 -3 -2 -3 -1  1 -4 -3 -2 11
Y -2 -2 -2 -3 -2 -1 -2 -3  2 -1 -1 -2 -1  3 -3 -2 -2  2  7
V  0 -3 -3 -3 -1 -2 -2 -3 -3  3  1 -2  1 -1 -2 -2  0 -3 -1  4
```

The percent identity is then calculated as:

$$\frac{\text{Total number of identical matches}}{[\text{length of the longer sequence plus the number of gaps introduced into the longer sequence in order to align the two sequences}]} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

**Conservative amino acid substitutions**

| | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

In addition to the 20 standard amino acids, non-standard amino acids (such as 4-hydroxyproline, 6-*N*-methyl lysine, 2-aminoisobutyric acid, isovaline and α -methyl serine) may be substituted for amino acid residues of the polypeptides of the present invention. A limited number of non-conservative amino acids, amino acids that are not encoded by the genetic code, and unnatural amino acids may be substituted for clostridial polypeptide amino acid residues. The polypeptides of the present invention can also comprise non-naturally occurring amino acid residues.

Non-naturally occurring amino acids include, without limitation, trans-3-methylproline, 2,4-methano-proline, cis-4-hydroxyproline, trans-4-hydroxyproline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an E. *coli* S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, *E. coli* cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by *in vitro* chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides of the present invention.

Essential amino acids in the polypeptides of the present invention can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992; Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides of the present invention.

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenised polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

**Examples**

A polypeptide is prepared for administration to a patient suffering from itch, providing a method for the suppression or treatment of itch. As defined in the present invention, the Targeting Moiety (TM) component of the prepared polypeptide binds to a G-protein coupled receptor , in particular to a Mrgpr G-protein coupled receptor, that is present on the itch-specific DRG neuron or a pruriceptor. Thereafter, the translocation component effects transport of the non-cytotoxic protease component of the polypeptide, which once inside the itch-specific DRG neuron or a pruriceptor, inhibits neurotransmitter secretion therefrom. Accordingly, the polypeptide reduces the level of neurotransmitter, and is capable of suppressing or treating itch.

Example 1

A 35 year-old female breast cancer patient suffers from debilitating chronic itch attributed to a drug reaction for the treatment of her malignancy. Treatment is by subcutaneous injection of 0.025 mg/kg of a polypeptide of the present invention comprising $BAM_{8-22}$ ligand (weekly injection over a period of six months), leading to a decrease in itch sensation and marked improvement in health. The patient reports effective itch relief.

Example 2

A 60 year-old male patient reports chronic itch and presents a severe case attributable to renal disease. A parenteral suspension of 0.07 mg/kg of a polypeptide of the present invention comprising a $SLIGRL-NH_2$ peptide is administered via laparoscopic duodenal injection (6 monthly injection regimen). Positive results including reduced itch are reported within the first month of treatment.

Example 3

A 37 year-old male patient with a family history of psoriasis suffers from chronic itch which is having a significant effect on his physical well-being and quality of life. Weekly dosage of 0.09 mg/kg of a polypeptide of the present invention comprising γ2-Melanocyte Stimulating Hormone (γ2-MSH) peptide is administered intravenously. The severity of psoriasis decreases and no further itch sensations are reported by the patient throughout the treatment period.

Example 4

A 45 year-old HIV patient suffering from a severe chronic itch is recently diagnosed with shingles, a disease having symptoms which often include extreme itchiness. After many sleepless nights of relentless scratching, she is prescribed monthly intravenous injections of 0.05 mg/kg of a polypeptide of the invention comprising chloroquine (CQ). The patient reports an improved rate of recovery, and no further sensations of itch after three months of treatment.

Example 5

A 6 year-old female patient with atopic eczema is prescribed a monthly oral dose of 0.1 mg/kg of an encapsulated formulation of a polypeptide of the invention comprising histamine HT1. The patient reports improvement in her condition after treatment, and disappearance of the red areas of the skin behind her knee.

Example 6

A 17 year-old male with Jorgen's syndrome reports symptoms of pruritus in his eyes, and skin rashes on the extremities. The clinician prescribes 0.001 mg/kg (local laparoscopic injection every 8 weeks) of a polypeptide of the invention comprising serotonin. In two months the patient reports a significant reduction in discomfort.

Example 7

An 18 year-old female patient is diagnosed with papular urticaria following a vacation to Africa. She presents a clinician with numerous lesions on exposed areas, particularly the lower legs but also arms, cheeks and waistline. Papules and post inflammatory scars are evident. Since diagnosis, the patient experiences a poorer overall quality of life. The patient is given treatment for her itch with monthly injections of 0.07 mg/kg of a polypeptide of the present invention comprising neuropeptides terminating in a NPFF/Y-G or a RF/Y-amide. During the six month treatment program, the patient reports no further itch sensations.

Example 8

A 59 year-old male patient with cutaneous B-cell lymphoma has subsequently developed red rash and dry skin with severe itch covering approximately 80% of the body. The patient's physician prescribes 0.1 mg/kg of a polypeptide of the invention comprising capsaicin, administered by laparoscopic duodenal injection every three months. After one dose of treatment, the patient reports no further itch.

Example 9

A 34 year-old female patient with polymorphic eruption of pregnancy (PEP) presents severe itch in the form of a rash with wheals and large inflamed areas of the skin on her lower abdomen, and limbs. The patient is treated by administering an adhesive patch onto the surface of his skin, which delivers the polypeptide of the invention comprising cortistatin via slow diffusion from the patch over a period of 2 weeks. The transdermal patch is replaced up until 12 weeks at the end of which the itch sensation is reported as disappeared.

**Animal model examples**

The polypeptides of the invention are tested as a treatment for itch, through the use of mouse models of itch-induced atopic dermatitis (AD) as described herein. BALB/c and C57BL/6 mice strains are equally suitable, as exemplified below.

Example 10

BALB/c mouse model of itch induced by repeated epicutaneous (EC) sensitization of tape-stripped skin with ovalbumin. The back skin of mice is shaved and tape stripped 6 times with 3M tape, mimicking skin injury inflicted by scratching in patients with atopic dermatitis (AD). 100 $\mu$g of OVA in 100 $\mu$l of normal saline is placed on a 1 x 1 cm patch of sterile gauze, which is secured to the skin with a transparent bio-occlusive dressing. This ensures that the antigen is not accessible to licking. Each mouse has a total of three one-week exposures to the patch at the same site that is separated from each other by 2 week intervals. EC sensitized mice develop increased scratching behavior and their skin develops lesions characterized by epidermal and dermal thickening.
A polypeptide of the invention is administered intradermally (i.d) into the back of the neck at 1h intervals at a concentration of 1mg/ml. Injection of PBS was used as a control.
'Itch' is measured by counting the number of 'bouts' of scratching prior to treatment with a polypeptide of the invention as compared with after treatment. A bout of scratching is defined as three or more individual rapid scratch movements with the hind paws to the area around the injection site (i.e. the back of the neck). Results show a marked reduction in scratching post-treatment with the polypeptide of the invention.

Example 11

BABL/c mice are subjected to EC application of the recombinant mite allergen Der p8 and exhibit features of dermatitis with epidermal hyperplasia and spongiosis. A polypeptide of the invention is administered as above. The findings are similar to those observed in the model of EC sensitization with OVA in that a significant reduction in scratching is noted after treatment with the polypeptide of the invention.

Example 12

BABL/c mice are exposed to *S. aureus* infection, known to exacerbate symptoms of AD and cause lesions in the skin. Results of administrating a polypeptide of the invention demonstrate a markedly reduced level of scratching by the subject treated with the polypeptide of the invention as compared with control mice. These results substantiate the use of polypeptides of the invention in the treatment of itch.

**Claims**

1.  A polypeptide, for use in suppressing or treating itch, wherein the polypeptide comprises:

    (i) a non-cytotoxic protease, which protease is capable of cleaving a SNARE protein in an itch-specific DRG neuron or a pruriceptor;
    (ii) a Targeting Moiety (TM) that is capable of binding to a Mas-related G protein-coupled receptor (Mrgpr) on the itch-specific DRG neuron or a pruriceptor, which Mrgpr is capable of undergoing endocytosis to be incorporated into an endosome within the itch-specific DRG neuron or a pruriceptor, and wherein said itch-specific DRG neuron or a pruriceptor expresses said SNARE protein; and
    (iii) a translocation domain that is capable of translocating the protease from within an endosome, across the endosomal membrane and into the cytosol of the itch-specific DRG neuron or a pruriceptor;

    with the proviso that the polypeptide is not a clostridial neurotoxin (holotoxin) molecule.

2.  The polypeptide for use according claim 1, wherein the TM binds to a receptor selected from the group comprising MrgprX, MrgprA, or MrgprC or receptor analogues thereof.

3.  The polypeptide for use according to claim 1 or 2 wherein the TM is selected from the group consisting of: a Bovine Adrenal Medulla (BAM) peptide, a Melanocyte Stimulating Hormone peptide (MSH), neuropeptides terminating in Y-G or Y-amide, a chloroquine (CQ), a peptide comprising $SLIGRLNH_2$, histamine, serotonin, capsaicin, cortistatin or truncations or peptide analogues thereof.

4.  The polypeptide for use according to any of the previous claims wherein the TM is selected from the group comprising: $BAM_{8-22}$, a $\gamma$2-MSH, SLIGRL, NPAF, NPFF, a chloroquine (CQ), a histamine, or serotonin, capsaicin, cortistatin or truncations or peptide analogues thereof.

5.  The polypeptide for use according to any of the previous claims wherein the TM binds to the MrgprX1 receptor.

6.  The polypeptide for use according to any of the previous claims wherein the TM is $BAM_{8-22}$ peptide or a truncation or peptide analogue thereof.

7.  The polypeptide for use according to any preceding claim, wherein the itch is histamine-independent itch.

8.  The polypeptide for use according to any preceding claim, wherein the use of an analgesic causes, or worsens the itch.

9.  The polypeptide for use according to claim 8, wherein said analgesic is an opioid pain reliever.

10. The polypeptide for use according to any preceding claim, wherein the itch is chronic itch, preferably chronic itch resulting from a renal disease, a liver disease, cancer or a skin disease.

11. A polypeptide for use according to any preceding claim, wherein the non-cytotoxic protease comprises a clostridial neurotoxin L-chain or an IgA protease

12. A polypeptide for use according to any preceding claim, wherein the translocation domain comprises a clostridial neurotoxin translocation domain.

13. A nucleic acid encoding a polypeptide according to any of the previous claims for use in suppressing or treating itch.

14. A polypeptide comprising:

(i) a non-cytotoxic protease, which protease is capable of cleaving a SNARE protein in an itch-specific DRG neuron or a pruriceptor;

(ii) a Targeting Moiety (TM) that is capable of binding to a Mas-related G protein-coupled receptor (Mrgpr) on the itch-specific DRG neuron or a pruriceptor, which Mrgpr is capable of undergoing endocytosis to be incorporated into an endosome within the itch-specific DRG neuron or a pruriceptor, and wherein said itch-specific DRG neuron or a pruriceptor expresses said SNARE protein; and

(iii) a translocation domain that is capable of translocating the protease from within an endosome, across the endosomal membrane and into the cytosol of the itch-specific DRG neuron or a pruriceptor;

with the proviso that the polypeptide is not a clostridial neurotoxin (holotoxin) molecule, and wherein the TM is selected from the group consisting of: a Melanocyte Stimulating Hormone peptide (MSH), neuropeptides terminating in Y-G or Y-amide, a chloroquine (CQ), a peptide comprising SLIGRL-NH$_2$, histamine, serotonin, or truncations or peptide analogues thereof.

15. A nucleic acid encoding a polypeptide according to claim 14.


**Patentansprüche**

1. Polypeptid, zur Verwendung bei der Suppression oder Behandlung von Juckreiz, wobei das Polypeptid Folgendes umfasst:

(i) eine nicht-cytotoxische Protease, welche Protease zum Spalten eines SNARE-Proteins in einem juckreizspezifischen DRG-Neuron (Spinalganglion-Neuron) oder einem Prurizeptor fähig ist;

(ii) einen zielgerichteten Teil (TM, Targeting Moiety), der zur Bindung an einen Mas-verwandten G-Protein-gekoppelten Rezeptor (Mrgpr) auf dem juckreizspezifischen DRG-Neuron oder einem Prurizeptor fähig ist, welcher Mrgpr der Endocytose unterzogen werden kann, um in ein Endosom in dem juckreizspezifischen DRG-Neuron oder einem Prurizeptor inkorporiert werden zu können, und wobei das genannte juckreizspezifische DRG-Neuron oder ein Prurizeptor das genannte SNARE-Protein exprimiert; und

(iii) eine Translokationsdomäne, die zur Translokation der Protease aus dem Inneren eines Endosoms, über die Endosomenmembran und in das Cytosol des juckreizspezifischen DRG-Neurons oder eines Prurizeptors fähig ist;

mit der Maßgabe, dass das Polypeptid kein Clostridienneurotoxin-(Holotoxin)-Molekül ist.

2. Polypeptid zur Verwendung nach Anspruch 1, wobei der TM an einen Rezeptor bindet, der aus der Gruppe ausgewählt ist, umfassend MrgprX, MrgprA oder MrgprC oder Rezeptor-Analoga davon.

3. Polypeptid zur Verwendung nach Anspruch 1 oder 2, wobei der TM aus der Gruppe ausgewählt ist, bestehend aus: einem Rindernebennierenmark-(BAM, Bovine Adrenal Medulla)-Peptid, einem Melanocyten-stimulierenden Hormonpeptid (MSH), in Y-G- oder Y-Amid terminierenden Neuropeptiden, einem Chloroquin (CQ), einem SLIGRL-NH$_2$ umfassenden Peptid, Histamin, Serotonin, Capsaicin, Cortistatin oder Trunkationen oder Peptidanaloga davon.

4. Polypeptid zur Verwendung nach einem der vorangehenden Ansprüche, wobei der TM aus der Gruppe ausgewählt ist, bestehend aus: BAM$_{8-22}$, einem $\gamma$2-MSH, SLIGRL, NPAF, NPFF, einem Chloroquin (CQ), einem Histamin oder Serotonin, Capsaicin, Cortistatin oder Trunkationen oder Peptidanaloga davon.

5. Polypeptid zur Verwendung nach einem der vorangehenden Ansprüche, wobei der TM an den MrgprX1-Rezeptor bindet.

6. Polypeptid zur Verwendung nach einem der vorangehenden Ansprüche, wobei der TM ein BAM$_{8-22}$-Peptid oder eine Trunkation oder ein Peptidanalogon davon ist.

7. Polypeptid zur Verwendung nach einem der vorangehenden Ansprüche, wobei der Juckreiz ein Histaminunabhängiger Juckreiz ist.

8. Polypeptid zur Verwendung nach einem der vorangehenden Ansprüche, wobei die Verwendung eines Analgetikums den Juckreiz verursacht oder verschlimmert.

9. Polypeptid zur Verwendung nach Anspruch 8, wobei das genannte Analgetikum ein Opioid-Schmerzmittel ist.

10. Polypeptid zur Verwendung nach einem der vorangehenden Ansprüche, wobei der Juckreiz ein chronischer Juckreiz, bevorzugt ein chronischer Juckreiz infolge einer Nierenerkrankung, einer Lebererkrankung, von Krebs oder einer Hauterkrankung ist.

11. Polypeptid zur Verwendung nach einem der vorangehenden Ansprüche, wobei die nicht-cytotoxische Protease eine Clostridienneurotoxin-L-Kette oder eine IgA-Protease umfasst.

12. Polypeptid zur Verwendung nach einem der vorangehenden Ansprüche, wobei die Translokationsdomäne eine Clostridienneurotoxin-Translokationsdomäne umfasst.

13. Nukleinsäure, die für ein Polypeptid nach einem der vorangehenden Ansprüche zur Verwendung bei der Suppression oder Behandlung von Juckreiz kodiert.

14. Polypeptid, umfassend:

(i) eine nicht-cytotoxische Protease, welche Protease zum Spalten eines SNARE-Proteins in einem juckreizspezifischen DRG-Neuron (Spinalganglion-Neuron) oder einem Prurizeptor fähig ist;
(ii) einen zielgerichteten Teil (TM, Targeting Moiety), der zur Bindung an einen Mas-verwandten G-Protein-gekoppelten Rezeptor (Mrgpr) auf dem juckreizspezifischen DRG-Neuron oder einem Prurizeptor fähig ist, welcher Mrgpr der Endocytose unterzogen werden kann, um in ein Endosom in dem juckreizspezifischen DRG-Neuron oder einem Prurizeptor inkorporiert werden zu können, und wobei das genannte juckreizspezifische DRG-Neuron oder ein Prurizeptor das genannte SNARE-Protein exprimiert; und
(iii) eine Translokationsdomäne, die zur Translokation der Protease aus dem Inneren eines Endosoms, über die Endosomenmembran und in das Cytosol des juckreizspezifischen DRG-Neurons oder eines Prurizeptors fähig ist;

mit der Maßgabe, dass das Polypeptid kein Clostridienneurotoxin-(Holotoxin)-Molekül ist, und wobei der TM aus der Gruppe ausgewählt ist, bestehend aus: einem Melanocyten-stimulierenden Hormonpeptid (MSH), in Y-G- oder Y-Amid terminierenden Neuropeptiden, einem Chloroquin (CQ), einem SLIGRL-NH$_2$ umfassenden Peptid, Histamin, Serotonin oder Trunkationen oder Peptidanaloga davon.

15. Nukleinsäure, die für ein Polypeptid nach Anspruch 14 kodiert.

**Revendications**

1. Polypeptide, destiné à une utilisation dans la suppression ou le traitement des démangeaisons, le polypeptide comprenant :

(i) une protéase non cytotoxique, ladite protéase étant capable de cliver une protéine SNARE dans un neurone de ganglion rachidien spécifique des démangeaisons ou un pruricepteur ;
(ii) une fraction de ciblage (Targeting Moiety, TM) qui est capable de se lier à un récepteur couplé aux protéines G apparenté à Mas (Mrgpr) sur le neurone de ganglion rachidien spécifique des démangeaisons ou à un pruricepteur, ledit Mrgpr étant capable de subir une endocytose pour être incorporé dans un endosome à l'intérieur du neurone de ganglion rachidien spécifique des démangeaisons ou d'un pruricepteur, et ledit neurone de ganglion rachidien spécifique des démangeaisons ou pruricepteur exprimant ladite protéine SNARE ; et
(iii) un domaine de translocation qui est capable de transloquer la protéase à partir de l'intérieur d'un endosome, à travers la membrane endosomale et dans le cytosol du neurone de ganglion rachidien spécifique des démangeaisons ou un pruricepteur ;

sous réserve que le polypeptide ne soit pas une molécule de neurotoxine clostridienne (holotoxine).

2. Polypeptide destiné à une utilisation selon la revendication 1, dans lequel la TM se lie à un récepteur sélectionné dans le groupe comprenant MrgprX, MrgprA ou MrgprC ou des récepteurs analogues de ceux-ci.

3. Polypeptide destiné à une utilisation selon la revendication 1 ou 2, dans lequel la TM est sélectionnée dans le groupe constitué de : un peptide de médullosurrénale bovine (BAM), un peptide d'hormone mélano-stimulante (MSH), des neuropeptides à terminaisons Y-G ou Y-amide, une chloroquine (CQ), un peptide comprenant SLIGRL-NH$_2$, une histamine, une sérotonine, une capsaïcine, une cortistatine, ou des troncatures ou des peptides analogues de ceux-ci.

**4.** Polypeptide destiné à une utilisation selon l'une quelconque des revendications précédentes, dans lequel la TM est sélectionnée dans le groupe comprenant : BAM$_{8-22}$, une γ2-MSH, SLIGRL, NPAF, NPFF, une chloroquine (CQ), une histamine, ou une sérotonine, une capsaïcine, une cortistatine, ou des troncatures ou des peptides analogues de ceux-ci.

**5.** Polypeptide destiné à une utilisation selon l'une quelconque des revendications précédentes, dans lequel la TM se lie au récepteur MrgprX1.

**6.** Polypeptide destiné à une utilisation selon l'une quelconque des revendications précédentes, dans lequel la TM est un peptide BAM$_{8-22}$ ou une troncature ou un peptide analogue de celui-ci.

**7.** Polypeptide destiné à une utilisation selon l'une quelconque des revendications précédentes, la démangeaison étant une démangeaison indépendante de l'histamine.

**8.** Polypeptide destiné à une utilisation selon l'une quelconque des revendications précédentes, l'utilisation d'un analgésique provoquant ou aggravant la démangeaison.

**9.** Polypeptide destiné à une utilisation selon la revendication 8, ledit analgésique étant un opioïde antidouleur.

**10.** Polypeptide destiné à une utilisation selon l'une quelconque des revendications précédentes, la démangeaison étant une démangeaison chronique, préférablement une démangeaison chronique résultant d'une maladie rénale, d'une maladie du foie, d'un cancer ou d'une maladie de la peau.

**11.** Polypeptide destiné à une utilisation selon l'une quelconque des revendications précédentes, dans lequel la protéase non cytotoxique comprend une chaîne légère de neurotoxine clostridienne ou une protéase d'IgA.

**12.** Polypeptide destiné à une utilisation selon l'une quelconque des revendications précédentes, dans lequel le domaine de translocation comprend un domaine de translocation de neurotoxine clostridienne.

**13.** Acide nucléique codant un polypeptide selon l'une quelconque des revendications précédentes, destiné à une utilisation dans la suppression ou le traitement des démangeaisons.

**14.** Polypeptide comprenant :

(i) une protéase non cytotoxique, ladite protéase étant capable de cliver une protéine SNARE dans un neurone de ganglion rachidien spécifique des démangeaisons ou un pruricepteur ;
(ii) une fraction de ciblage (Targeting Moiety, TM) qui est capable de se lier à un récepteur couplé aux protéines G apparenté à Mas (Mrgpr) sur le neurone de ganglion rachidien spécifique des démangeaisons ou à un pruricepteur, ledit Mrgpr étant capable de subir une endocytose pour être incorporé dans un endosome à l'intérieur du neurone de ganglion rachidien spécifique des démangeaisons ou d'un pruricepteur, et ledit neurone de ganglion rachidien spécifique des démangeaisons ou pruricepteur exprimant ladite protéine SNARE ; et
(iii) un domaine de translocation qui est capable de transloquer la protéase à partir de l'intérieur d'un endosome, à travers la membrane endosomale et dans le cytosol du neurone de ganglion rachidien spécifique des démangeaisons ou un pruricepteur ;

sous réserve que le polypeptide ne soit pas une molécule de neurotoxine clostridienne (holotoxine), et dans lequel la TM est sélectionnée dans le groupe constitué de : un peptide d'hormone mélano-stimulante (MSH), des neuropeptides à terminaisons Y-G ou Y-amide, une chloroquine (CQ), un peptide comprenant SLIGRL-NH$_2$, une histamine, une sérotonine, ou des troncatures ou des peptides analogues de ceux-ci.

**15.** Acide nucléique codant un polypeptide selon la revendication 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9958571 A
- WO 99032272 A
- WO 2011022357 A
- WO 9421300 A
- WO 9633273 A
- WO 9807864 A
- WO 0010598 A
- WO 0121213 A
- WO 06059093 A
- WO 0062814 A
- WO 0004926 A
- WO 9315766 A

- WO 0061192 A
- EP 0257742 A
- WO 2012156743 A
- US 20070166332 A
- WO 2010094905 A
- WO 200244199 A
- WO 2007106115 A
- WO 2007104567 A
- WO 08008803 A
- WO 08008805 A
- US 5223409 A, Ladner
- WO 9206204 A

### Non-patent literature cited in the description

- **HERMANSON, G.T.** Bioconjugate techniques. Academic Press, 1996
- **WONG, S.S.** Chemistry of protein conjugation and cross-linking. CRC Press, 1991
- **NAGY et al.** *PNAS,* 1998, vol. 95, 1794-99
- **NORD, K. et al.** Binding proteins selected from combinatorial libraries of an alpha-helical bacterial receptor domain. *Nat Biotechnol,* 1997, vol. 15, 772-777
- **SKERRA.** Alternative binding proteins: anticalins - harnessing the structural plasticity of the lipocalin ligand pocket to engineer novel binding activities. *FEBS J.,* 2008, vol. 275, 2677-83
- **DINEEN et al.** The Adnectin CT-322 is a novel VEGF receptor 2 inhibitor that decreases tumour burden in an orthotropic mouse model of pancreatic cancer. *BMC Cancer,* 2008, vol. 8, 352
- **SILVERMAN et al.** Multivalent avimer proteins evolved by exon shuffling of a family of human receptor domains. *Nat Biotechnol,* 2005, vol. 23, 1556-61
- **ZAHND et al.** Selection and characterization of Her2 binding-designed ankyrin repeat proteins. *J Biol Chem.,* 2006, vol. 281, 35167-75
- Receptor-binding studies, a brief outline. **HULME, E.C.** Receptor biochemistry, A Practical Approach. Oxford University Press, 1990, 303-311
- **RUMMEL et al.** *Molecular Microbiol.,* 2004, vol. 51, 631-634
- **AHMED, S.A.** *Protein J.,* 2008
- **POHLNER, J. et al.** *Nature,* 1987, vol. 325, 458-462

- **CHEN ; BARBIERI.** *PNAS,* 2009, vol. 106 (23), 9180-9184
- **SHONE C.** *Eur. J. Biochem,* 1987, vol. 167 (1), 175-180
- **BLAUSTEIN.** *FEBS Letts,* 1987, vol. 226 (1), 115-120
- Membrane Fusion Techniques. Methods in Enzymology. Academic Press, 1993, vol. 220-221
- **HENDERSON et al.** The Clostridia: Molecular Biology and Pathogenesis. Academic press, 1997
- **O=KEEFE et al.** *Proc. Natl. Acad. Sci. USA,* 1992, vol. 89, 6202-6206
- **SILVERMAN et al.** *J. Biol. Chem.,* 1993, vol. 269, 22524-22532
- **LONDON, E.** *Biochem. Biophys. Acta.,* 1992, vol. 1112, 25-51
- **PRIOR et al.** *Biochemistry,* 1992, vol. 31, 3555-3559
- **BLANKE et al.** *Proc. Natl. Acad. Sci. USA,* 1996, vol. 93, 8437-8442
- **PLANK et al.** *J. Biol. Chem.,* 1994, vol. 269, 12918-12924
- **WAGNER et al.** *PNAS,* 1992, vol. 89, 7934-7938
- **MURATA et al.** *Biochem.,* 1992, vol. 31, 1986-1992
- **SILVERMAN et al.** *J. Biol. Chem.,* 1994, vol. 269, 22524-22532
- **LONDON E.** *Biochem. Biophys. Acta.,* 1992, vol. 1113, 25-51
- **KIHARA ; PASTAN.** *Bioconj Chem.,* 1994, vol. 5, 532-538

- **MURATA et al.** *Biochemistry,* 1992, vol. 31, 1986-1992
- **KIELIAN et al.** *J Cell Biol.,* 1996, vol. 134 (4), 863-872
- **YAO et al.** *Virology,* 2003, vol. 310 (2), 319-332
- **SETH et al.** *J Virol,* 2003, vol. 77 (11), 6520-6527
- **PICARD-MAUREAU et al.** *J Virol.,* 2003, vol. 77 (8), 4722-4730
- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research,* 1994, vol. 22 (22), 4673-4680
- **OSAMU GOTOH.** Significant Improvement in Accuracy of Multiple Protein. Sequence Alignments by Iterative Refinement as Assessed by Reference to Structural Alignments. *J. Mol. Biol.,* 1996, vol. 264 (4), 823-838
- **ERIC DEPIEREUX ; ERNEST FEYTMANS.** Match-Box: A Fundamentally New Algorithm for the Simultaneous Alignment of Several Protein Sequences. *CABIOS,* 1992, vol. 8 (5), 501-509
- **C. E. LAWRENCE et al.** Detecting Subtle Sequence Signals: A Gibbs Sampling Strategy for Multiple Alignment. *Science,* 1993, vol. 262 (5131), 208-214
- **IVO VAN WALLE et al.** Align-M - A New Algorithm for Multiple Alignment of Highly Divergent Sequences. *Bioinformatics,* 2004, vol. 20 (9), 1428-1435
- **ALTSCHUL et al.** *Bull. Math. Bio.,* 1986, vol. 48, 603-16
- **HENIKOFF ; HENIKOFF.** *Proc. Natl. Acad. Sci. USA,* 1992, vol. 89, 10915-19
- **ROBERTSON et al.** *J. Am. Chem. Soc.,* 1991, vol. 113, 2722
- **ELLMAN et al.** *Methods Enzymol.,* 1991, vol. 202, 301
- **CHUNG et al.** *Science,* 1993, vol. 259, 806-9
- **CHUNG et al.** *Proc. Natl. Acad. Sci. USA,* 1993, vol. 90, 10145-9
- **TURCATTI et al.** *J. Biol. Chem.,* 1996, vol. 271, 19991-8
- **KOIDE et al.** *Biochem.,* 1994, vol. 33, 7470-6
- **WYNN ; RICHARDS.** *Protein Sci.,* 1993, vol. 2, 395-403
- **CUNNINGHAM ; WELLS.** *Science,* 1989, vol. 244, 1081-5
- **DE VOS et al.** *Science,* 1992, vol. 255, 306-12
- **SMITH et al.** *J. Mol. Biol.,* 1992, vol. 224, 899-904
- **WLODAVER et al.** *FEBS Lett.,* 1992, vol. 309, 59-64
- **OLSON ; SAUER.** *Science,* 1988, vol. 241, 53-7
- **BOWIE ; SAUER.** *Proc. Natl. Acad. Sci. USA,* 1989, vol. 86, 2152-6
- **LOWMAN et al.** *Biochem.,* 1991, vol. 30, 10832-7
- **DERBYSHIRE et al.** *Gene,* 1986, vol. 46, 145
- **NER et al.** *DNA,* 1988, vol. 7, 127